# EUROPEAN PATENT APPLICATION

(11) **EP 4 582 530 A1**
(43) Date of publication of application: **09.07.2025**
(21) Application number: 23860556.2
(22) Date of filing: 04.09.2023
(51) Int. Cl.: C12M 1/04, C12M 1/34, C12N 1/00, C12N 1/20

(54) **CULTURE DEVICE AND METHOD FOR CONTROLLING CULTURE DEVICE**

(30) Priority: 02.09.2022 JP 2022140319; 31.07.2023 JP 2023125055
(71) Applicant: FUJIFILM Corporation, Tokyo 106-8620 (JP)
(72) Inventor: WAKITA, Taku, Ashigarakami-gun, Kanagawa 258-8577 (JP); FUKUNAGA, Kazuto, Ashigarakami-gun, Kanagawa 258-8577 (JP); YOSHIDA, Tetsuya, Ashigarakami-gun, Kanagawa 258-8577 (JP); YAMAZAKI, Hidekazu, Ashigarakami-gun, Kanagawa 258-8577 (JP)
(74) Representative: Hoffmann Eitle
(86) International application number: PCT/JP2023/032206
(87) International publication number: WO 2024/048796

(57) **Abstract**

A culture device for culturing a microorganism or a cell that uses a raw material gas containing a flammable gas for proliferation or for production of an organic substance includes: a culture tank that accommodates a culture solution in which the microorganism or the cell is cultured; a raw material gas supply unit that supplies the raw material gas to the culture tank; a composition ratio measurement unit that measures a composition ratio of a mixed gas in a space present above a liquid surface of the culture solution in the culture tank; and a composition ratio control mechanism that supplies an adjustment gas to the space according to the measured composition ratio of the mixed gas, thereby maintaining the composition ratio of the mixed gas in the space at a target value outside a preset explosion range.

## Description

### BACKGROUND OF THE INVENTION

### 1. Field of the Invention

The technology of the present disclosure relates to a culture device and a method for controlling the culture device.

### 2. Description of the Related Art

In order to suppress greenhouse gas emissions, research on extrication from dependence on petroleum as a raw material is actively conducted worldwide. As part of this research, a technology of using a carbon source other than petroleum as a raw material has been developed in the field of manufacturing products such as chemical products and bioproducts. For example, microorganisms are known to use a carbon source other than petroleum as a raw material for proliferation or production of an organic substance. An example of such a microorganism is hydrogen-oxidizing bacteria. The hydrogen-oxidizing bacteria proliferate by taking in a gas containing carbon dioxide, hydrogen, and oxygen as a raw material, and further efficiently produce organic substances such as alcohol and amino acids by modifying genes of the hydrogen-oxidizing bacteria. The hydrogen-oxidizing bacteria are a general term for chemosynthetic bacteria that convert carbon contained in carbon dioxide into an organic substance by using energy generated by the oxidation of hydrogen, and take the organic substance into the bacteria. The hydrogen-oxidizing bacteria are attracting attention also from the viewpoint that carbon dioxide is a raw material for proliferation or production of an organic substance, and thus carbon dioxide emitted as a greenhouse gas can be effectively used.

JP1976-038480A (JP-S51-038480A) and JP2564008B disclose a culture device for culturing hydrogen-oxidizing bacteria. The culture device includes a culture tank that accommodates a culture solution. A raw material gas containing carbon dioxide, hydrogen, and oxygen is supplied into the culture tank, and a part of the carbon dioxide, the hydrogen, and the oxygen contained in the raw material gas is dissolved in the culture solution and is consumed for the proliferation of the hydrogen-oxidizing bacteria or the production of the organic substance. The remaining raw material gas is released into a space present above a liquid surface of the culture solution in the culture tank and is exhausted to the outside of the culture tank. Since a mixed gas containing hydrogen, which is a flammable gas, and oxygen has a risk of explosion depending on a composition ratio, JP1976-038480A (JP-S51-038480A) and JP2564008B disclose a technology for suppressing the explosion.

In JP1976-038480A (JP-S51-038480A), a hydrogen tank that accommodates hydrogen and an oxygen tank that accommodates oxygen are each independently provided separately from the culture tank that accommodates the culture solution, and the culture solution is circulated between the three tanks of the culture tank, the hydrogen tank, and the oxygen tank. In JP1976-038480A (JP-S51-038480A), since the hydrogen tank and the oxygen tank are independently present, the generation of the mixed gas containing hydrogen and oxygen, which causes an explosion, is suppressed.

In JP2564008B, after the supply of oxygen contained in the raw material gas is made independently controllable, a dissolved oxygen concentration in the culture solution is measured, and a supply amount of oxygen to the culture tank is restricted based on the measured dissolved oxygen concentration.

### SUMMARY OF THE INVENTION

In the method described in JP1976-038480A (JP-S51-038480A), in a circulation path of the culture solution, the culture solution passes through the hydrogen tank in which hydrogen is rich and the oxygen tank in which oxygen is rich. In order to efficiently culture microorganisms such as hydrogen-oxidizing bacteria, it is preferable to constantly maintain concentrations of hydrogen and oxygen in the culture solution at appropriate concentrations. However, in the hydrogen tank and the oxygen tank, one of hydrogen and oxygen is rich, and thus the concentration thereof cannot be maintained at an appropriate concentration while the culture solution passes through the hydrogen tank and the oxygen tank. Therefore, there was a concern in JP1976-038480A (JP-S51-038480A) from the viewpoint of culture efficiency of microorganisms.

In the method described in JP2564008B, the dissolved oxygen concentration in the culture solution is measured, but no measures have been taken for the composition ratio of the mixed gas released from the culture solution. Since the composition ratio of the mixed gas that causes the explosion is not controlled, there is a concern from the viewpoint of safety in JP2564008B in terms of preventing the explosion. In addition, in JP2564008B, from the viewpoint of preventing the explosion, the dissolved oxygen concentration in the culture solution is controlled by restricting the supply amount of oxygen. Therefore, the supply amount of oxygen necessary for the proliferation of microorganisms or the production of organic substances may be reduced, which is a concern from the viewpoint of the culture efficiency of microorganisms.

The technology of the present disclosure provides a culture device and a method for controlling the culture device, which can improve culture efficiency and safety as compared with the related art in a case of culturing a microorganism or a cell that uses a raw material gas containing a flammable gas for proliferation or for production of an organic substance.

In order to achieve the above object, according to an aspect of the technology of the present disclosure, there is provided a culture device for culturing a microorganism or a cell that uses a raw material gas containing a flammable gas for proliferation or for production of an organic substance, the culture device comprising: a culture tank that accommodates a culture solution in which the microorganism or the cell is cultured; a raw material gas supply unit that supplies the raw material gas to the culture tank; a composition ratio measurement unit that measures a composition ratio of a mixed gas in a space present above a liquid surface of the culture solution in the culture tank; and a composition ratio control mechanism that supplies an adjustment gas to the space according to the measured composition ratio of the mixed gas, thereby maintaining the composition ratio of the mixed gas in the space at a target value outside a preset explosion range.

It is preferable that the adjustment gas is an inert gas.

It is preferable that the target value outside the explosion range is set to an oxygen concentration of less than 5% or a hydrogen concentration of less than 4%, and the composition ratio control mechanism controls the composition ratio based on the target value.

It is preferable that the target value outside the explosion range is an oxygen concentration of 5% or more and 9% or less and a water vapor concentration of 7% or more, and the composition ratio control mechanism controls the composition ratio based on the target value.

It is preferable that the culture device further comprises a temperature control mechanism that controls a temperature of the culture solution.

It is preferable that the temperature control mechanism controls the temperature of the culture solution to 40°C or higher.

It is preferable that the culture device further comprises a scattering mechanism that scatters a liquid containing water into the space.

It is preferable that the liquid is an aqueous solution containing at least a part of components of the culture solution.

It is preferable that the culture device further comprises a foam layer forming portion that forms a foam layer on the liquid surface of the culture solution.

It is preferable that the culture device further comprises a circulation path for reusing the mixed gas in the space as the raw material gas.

It is preferable that the flammable gas is hydrogen.

It is preferable that the raw material gas contains hydrogen, oxygen, and carbon dioxide.

It is preferable that the adjustment gas is carbon dioxide.

It is preferable that the microorganism is a hydrogen-oxidizing bacterium.

It is preferable that the culture device further comprises: a pressure measurement unit that measures a pressure inside the space; and a pressure control mechanism that controls the pressure by adjusting at least one of a supply amount of the raw material gas, a discharge amount of the mixed gas, or a supply amount of the adjustment gas, based on the measured pressure.

It is preferable that the culture device further comprises: a dissolved gas concentration measurement unit that measures a dissolved gas concentration of the raw material gas dissolved in the culture solution; and a raw material gas control mechanism that controls a supply amount of the raw material gas according to the dissolved gas concentration.

It is preferable that the dissolved gas concentration measurement unit measures a concentration of each component contained in the raw material gas, and the raw material gas control mechanism controls the supply amount for each component.

According to another aspect of the technology of the present disclosure, there is provided a method for controlling a culture device for culturing a microorganism or a cell that uses a raw material gas containing a flammable gas for proliferation or for production of an organic substance, the culture device including a culture tank that accommodates a culture solution in which the microorganism or the cell is cultured, and a raw material gas supply unit that supplies the raw material gas to the culture tank, the method comprising: measuring a composition ratio of a mixed gas in a space present above a liquid surface of the culture solution in the culture tank; and supplying an adjustment gas to the space according to the measured composition ratio of the mixed gas, thereby maintaining the composition ratio of the mixed gas in the space at a target value outside a preset explosion range.

According to the technology of the present disclosure, in a case of culturing a microorganism or a cell that uses a raw material gas containing a flammable gas for proliferation or for production of an organic substance, it is possible to improve culture efficiency and safety as compared with the related art.

### BRIEF DESCRIPTION OF THE DRAWINGS

Fig. 1 is a diagram illustrating a function of a culture device.
Fig. 2 is a configuration diagram of a culture device according to a first embodiment.
Fig. 3 is an explanatory diagram of information in a memory.
Fig. 4 is a flowchart of a composition ratio control process of a mixed gas.
Fig. 5 is a configuration diagram of a culture device according to a second embodiment.
Fig. 6 is a diagram showing a specific configuration of a dissolved gas concentration measurement unit.
Fig. 7 is a flowchart of a dissolved gas concentration adjustment process.
Fig. 8 is a diagram showing an overall configuration of a culture device according to a third embodiment.
Fig. 9 is a diagram showing a configuration in which a raw material gas is supplied for each component.
Fig. 10 is a diagram showing a configuration for recovering and reusing a culture solution.
Fig. 11 is a diagram showing a configuration for separating a specific component from the culture solution.
Fig. 12 is a diagram showing a disposition of an extraction port of the culture solution.
Fig. 13 is a diagram showing an index of a bubble diameter.
Fig. 14 is a diagram showing a surface tension depressant.
Fig. 15 is a diagram showing a configuration for reusing a waste gas.

### DESCRIPTION OF THE PREFERRED EMBODIMENTS

### [First Embodiment]

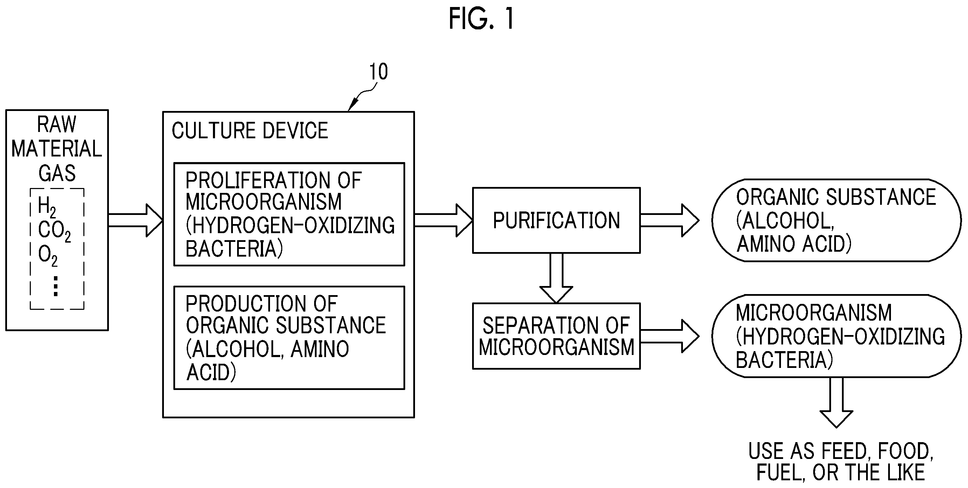

As shown in Fig. 1, a culture device 10 according to an embodiment of the technology of the present disclosure cultures hydrogen-oxidizing bacteria as microorganisms, as an example. A raw material for proliferating the hydrogen-oxidizing bacteria contains an inorganic component such as ammonium sulfate in addition to hydrogen (H₂), oxygen (O₂), and carbon dioxide (CO₂). The hydrogen is an example of a flammable gas. The culture device 10 supplies a raw material gas to a culture solution to proliferate the hydrogen-oxidizing bacteria in the culture solution. In addition, in the culture solution, the hydrogen-oxidizing bacteria efficiently produce organic substances such as alcohol and amino acids by modifying genes of the hydrogen-oxidizing bacteria. The culture solution is purified to extract an organic substance. The extracted organic substance is used, for example, in the production of a chemical product, a bioproduct, or the like. In addition, the hydrogen-oxidizing bacteria are separated from the culture solution, and the separated hydrogen-oxidizing bacteria are considered for use as, for example, feed, food, fuel, or the like.

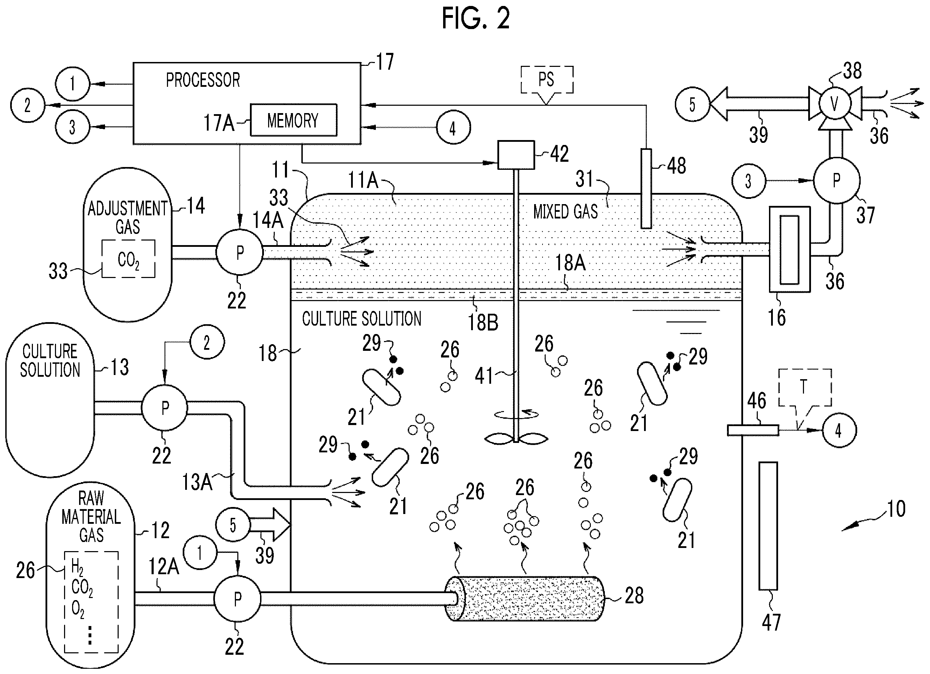

As shown in Fig. 2, the culture device 10 comprises a culture tank 11, a raw material gas tank 12, a culture solution tank 13, an adjustment gas tank 14, a composition ratio measurement unit 16, and a processor 17. The culture tank 11 accommodates a culture solution 18. Examples of the hydrogen-oxidizing bacteria 21 to be cultured by the culture device 10 include bacteria described in JP6528295B, such as the genus Hydrogenophilus bacteria. In this case, as described in JP6528295B, the culture solution 18 is, for example, an aqueous solution obtained by dissolving ammonium sulfate ((NH₄)₂SO₄), potassium dihydrogen phosphate (KH₂PO₄), dipotassium hydrogen phosphate (K₂HPO₄), sodium chloride (NaCl), or the like in water. The culture tank 11 is an example of a "culture tank" according to the technology of the present disclosure.

Supply paths 12A, 13A, and 14A of the raw material gas tank 12, the culture solution tank 13, and the adjustment gas tank 14 are connected to the culture tank 11. Each of the supply paths 12A, 13A, and 14A is composed of a pipe, a valve, and the like. In addition, a pump 22 is disposed on each of the supply paths 12A, 13A, and 14A.

The culture solution 18 is accommodated in the culture solution tank 13. The culture solution 18 is supplied from the culture solution tank 13 to the culture tank 11 through the supply path 13A by driving the pump 22 on the supply path 13A. A supply timing and a supply amount of the culture solution 18 are controlled by the processor 17 that controls the driving of the pump 22.

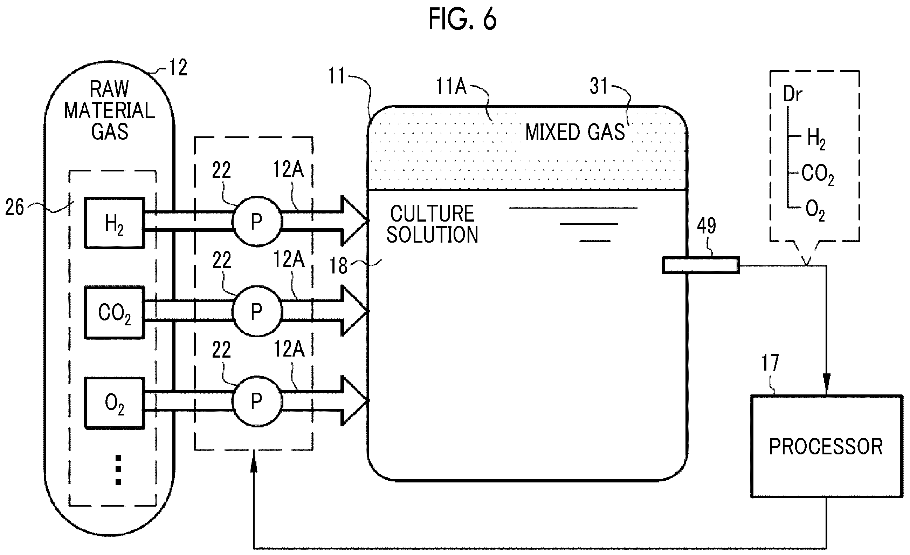

A raw material gas 26 is accommodated in the raw material gas tank 12. As described above, the raw material gas 26 is, for example, a gas containing each component such as hydrogen (H₂), oxygen (O₂), and carbon dioxide (CO₂). The raw material gas 26 is supplied from the raw material gas tank 12 to the culture tank 11 through the supply path 12A by driving the pump 22 on the supply path 12A. A supply timing and a supply amount of the raw material gas 26 are controlled by the processor 17 that controls the driving of the pump 22. In the present example, the raw material gas 26 is supplied in a state in which a plurality of components are mixed, but the plurality of components may be individually supplied. In this case, the supply path and the pump are provided for each component (see, for example, Fig. 6).

A downstream end of the supply path 12A is disposed in the culture tank 11, and a sparger 28 is provided at the downstream end. The sparger 28 is provided for dispersing and supplying the raw material gas 26 into the culture solution 18. As the sparger 28, for example, a ventilation pipe formed of a porous material is used.

A part of the raw material gas 26 supplied into the culture solution 18 is dissolved in the culture solution 18. The dissolved raw material gas 26 is taken in by the hydrogen-oxidizing bacteria 21 and is consumed for the proliferation of the hydrogen-oxidizing bacteria 21 or the production of the organic substance 29. The organic substance 29 is, for example, alcohol or an amino acid. The raw material gas 26 that is not dissolved in the culture solution 18 is released into a space 11A present above a liquid surface 18A of the culture solution 18. The space 11A is filled with a mixed gas 31 which is the raw material gas 26 released without being dissolved in the culture solution 18. The raw material gas tank 12 and the pump 22 on the supply path 12A are examples of a "raw material gas supply unit".

An adjustment gas 33 is accommodated in the adjustment gas tank 14. The adjustment gas 33 is supplied from the adjustment gas tank 14 to the space 11A of the culture tank 11 through the supply path 14A by driving the pump 22 on the supply path 14A. A supply timing and a supply amount of the adjustment gas 33 are controlled by the processor 17 that controls the driving of the pump 22.

The mixed gas 31 in the space 11A includes hydrogen and oxygen, which are components of the raw material gas 26. The hydrogen is a flammable gas, and the adjustment gas 33 is used to maintain a composition ratio of the mixed gas 31 in the space 11A at a target value outside a preset explosion range. In the mixed gas 31, the hydrogen, which is a flammable gas, is mixed with the oxygen. In this case, an explosion phenomenon occurs depending on the composition ratio of the mixed gas 31. The composition ratio of the mixed gas in a case where an explosion phenomenon occurs is referred to as an explosion range. As will be described later, in the processor 17, a target value outside the explosion range is set. The target value outside the explosion range varies depending on components of the mixed gas 31. In a case where the mixed gas 31 is a mixed gas including hydrogen and oxygen as in the present example, the target value outside the explosion range is generally an oxygen concentration of less than 5% or a hydrogen concentration of less than 4% in the mixed gas 31.

Furthermore, the present applicant has verified through an experiment that, in a case where a water vapor concentration is 7% or more, the composition ratio of the mixed gas can be maintained outside the explosion range even in a case where the oxygen concentration is 5% or more. Therefore, in the present example, as an example, the target value outside the explosion range is set such that, in a case where the water vapor concentration is 7% or more and 90% or less, the oxygen concentration is set in a range of 5% or more and 9% or less.

The experiment was performed by the following method. As an explosion container, a stainless steel chamber having an inner diameter of 200 mm, a depth of 200 mm, and a volume of 6 L was used. The explosion container was filled with hydrogen, oxygen, carbon dioxide, and water vapor (water vapor was filled by pouring water into the explosion container and performing vacuuming up to a saturated water vapor pressure at a temperature inside the container to vaporize the water. The hydrogen, oxygen, and carbon dioxide were filled from a gas cylinder through a gas introduction pipe provided in the explosion container). After rotating a stirring fan inside the explosion container to homogenize the gas, ignition of the gas was attempted using a thin wire explosion device provided in the explosion container. The determination of ignition or non-ignition was performed based on the presence or absence of a pressure increase and the presence or absence of generation of a negative pressure inside the explosion container due to the generation of water. The pressure was measured by a pressure sensor (PHS-B-10MP manufactured by Kyowa Electronic Instruments Co., Ltd.) disposed on an upper valve of a gas discharge pipe provided in the explosion container.

Table 1 shows experimental results of whether or not ignition occurs depending on a gas concentration of the mixed gas. Both Example 1 and Comparative Example 1 in Table 1 are examples in which the water vapor concentration is 0%, and concentrations other than the oxygen concentration are almost the same. In Example 1 in which ignition was not made, the oxygen concentration was 4% which is less than 5%, and in Comparative Example 1 in which ignition was made, the oxygen concentration was 9% which exceeds 5%. As is generally known, experimental results of Example 1 and Comparative Example 1 show that, in a case where the water vapor concentration is 0%, ignition does not occur in a case where the oxygen concentration is less than 5%.

In addition, all of Example 2, Example 3, and Comparative Example 2 are examples in which the water vapor concentration is 7% or more, and only the oxygen concentration is significantly different. In Example 2 and Example 3, the oxygen concentration was 9%, and in Comparative Example 2, the oxygen concentration was 16% which exceeds 9%. From these experimental results, it was found that in a case where the water vapor concentration is 7% or more, the ignition does not occur even in a case where the oxygen concentration is 5% or more but 9% or less.

**[Table 1]**

| | Gas Concentration | | | | Temperature | Result |
|---|---|---|---|---|---|---|
| | H₂ | O₂ | CO₂ | H₂O | | |
| Example 1 | 72% | 4% | 24% | 0% | 31°C | Non-ignition |
| Example2 | 70% | 9% | 14% | 7% | 44°C | Non-ignition |
| Example3 | 66% | 9% | 13% | 12% | 52°C | Non-ignition |
| Comparative Example1 | 67% | 9% | 24% | 0% | 31°C | Ignition |
| Comparative Example2 | 59% | 16% | 13% | 12% | 52°C | Ignition |

In Table 1, there are no experimental results in a case where the hydrogen concentration is less than 4%, but it is known that in a case where the hydrogen concentration is less than 4% instead of the oxygen concentration being less than 5%, the mixed gas will not ignite even in a case where the water vapor concentration is 0%. Therefore, as the target value outside the explosion range, the hydrogen concentration of the mixed gas 31 may be less than 4% instead of the oxygen concentration of the mixed gas 31 being less than 5%.

The adjustment gas 33 is, for example, an inert gas. More specifically, in the present example, carbon dioxide (CO₂) is used. By supplying the adjustment gas 33 to the space 11A, the oxygen concentration or the hydrogen concentration decreases, and the composition ratio of the mixed gas 31 is maintained at the target value outside the explosion range. In addition to the inert gas, any gas can be used as the adjustment gas 33. For example, any gas may be used as the adjustment gas 33, such as hydrogen, which is a flammable gas, to set the oxygen concentration of the mixed gas 31 to less than 5%, so that the composition ratio of the mixed gas 31 is set to the target value outside the explosion range. As described above, any gas can be used as the adjustment gas 33, but as will be described later, in a case where the mixed gas 31 is reused, it is preferable to use the components of the raw material gas 26 as the adjustment gas 33.

The composition ratio measurement unit 16 measures the composition ratio of the mixed gas 31. The composition ratio measurement unit 16 is disposed on a discharge path 36 through which the mixed gas 31 is discharged from the space 11A of the culture tank 11. The composition ratio measurement unit 16 is composed of, for example, a gas analyzer that measures a hydrogen concentration (for example, model number TCA-51d manufactured by Horiba Ltd.), a gas analyzer that measures an oxygen concentration (for example, model number VA-5113 manufactured by Horiba Ltd.), a gas analyzer that measures a carbon dioxide concentration (for example, model number VA-5113 manufactured by Horiba Ltd.), and a flowmeter that measures a total flow rate of the mixed gas 31 (for example, model number CMFS007M manufactured by Emerson Electric Co.). As a result, the composition ratio measurement unit 16 can measure each component and the total flow rate of the mixed gas 31. The composition ratio measurement unit 16 outputs each measurement value to the processor 17.

The processor 17 determines a supply amount of the adjustment gas 33 such that the oxygen concentration of the mixed gas 31 is maintained below a preset upper limit value, for example, based on the measured values of the total flow rate and the oxygen concentration of the mixed gas 31. Then, the processor 17 controls the pump 22 to supply the determined supply amount of the adjustment gas 33 from the adjustment gas tank 14 to the space 11A. As a result, the oxygen concentration decreases, and the composition ratio of the mixed gas 31 in the space 11A is maintained at the target value outside the preset explosion range. The processor 17, the adjustment gas tank 14, and the pump 22 on the supply path 14A are examples of a "composition ratio control mechanism" according to the technology of the present disclosure.

In addition, a pump 37 for discharging the mixed gas 31 and a three-way valve 38 are provided on the discharge path 36. The mixed gas 31 is a gas obtained by mixing the adjustment gas 33 with the raw material gas 26 that has not been dissolved in the culture solution 18. Therefore, the mixed gas 31 can be reused as the raw material gas 26. Therefore, a circulation path 39 for returning the mixed gas 31 to the culture tank 11 via the three-way valve 38 is connected to the discharge path 36. The mixed gas 31 is resupplied to the culture tank 11 again as the raw material gas 26 by the circulation path 39. In this manner, the mixed gas 31 including the adjustment gas 33 is reused. The three-way valve 38 switches between a state in which the discharge path 36 communicates with the circulation path 39 and a state in which the communication between the discharge path 36 and the circulation path 39 is closed. The three-way valve 38 switches whether the mixed gas 31 is sent to the circulation path 39 or is discharged to the outside.

Although not shown in Fig. 2 for convenience, for example, a downstream end of the circulation path 39 is inserted into the culture tank 11, and the sparger 28 is provided at the downstream end of the circulation path 39 in the same manner as the supply path 12A.

In addition, a stirring rod 41 that stirs the culture solution 18 is provided in the culture tank 11. The stirring rod 41 is a support rod provided with a screw, and is provided such that the screw is disposed in the culture solution 18. The stirring rod 41 rotates with the support rod as a rotation shaft by driving a motor 42 controlled by the processor 17. As a result, the culture solution 18 is stirred. The stirring rod 41 stirs the culture solution 18 to diffuse the hydrogen-oxidizing bacteria and the raw material gas 26 in the culture solution 18 such that local uneven distribution does not occur.

In addition, the stirring rod 41 stirs the culture solution 18 to generate bubbles from the culture solution 18 and form a foam layer 18B on the liquid surface 18A of the culture solution 18. In a case where a liquid film such as the foam layer 18B is formed on the liquid surface 18A of the culture solution 18, a heat capacity of the space 11A increases, which contributes to the suppression of the explosion. The stirring rod 41 is an example of a "foam layer forming portion" according to the technology of the present disclosure.

In addition, the culture tank 11 is provided with a thermometer 46 that measures a temperature T in the culture solution 18, a temperature adjuster 47 that heats or cools the culture solution 18, and a pressure gauge 48 that measures a pressure PS in the space 11A. The pressure gauge 48 is an example of a "pressure measurement unit" according to the technology of the present disclosure. The culture solution 18 is maintained at a target temperature suitable for culturing the hydrogen-oxidizing bacteria. The target temperature is, for example, 40°C or higher and 95°C or lower. The target temperature is set for each microorganism. In a case where the hydrogen-oxidizing bacteria belongs to the genus Hydrogenophilus, the target temperature is more preferably about 50°C. The culture solution 18 is heated to the target temperature, and in some cases, cooling is required. This is because the culture solution 18 may exceed the target temperature due to heat generation during the proliferation of the microorganisms to be cultured. The processor 17 controls driving of the temperature adjuster 47 such that the temperature T of the culture solution 18 is maintained at the target temperature, based on the temperature T measured by the thermometer 46.

In addition, since both the culture solution 18 and the space 11A are present in the culture tank 11, in a case where the temperature T of the culture solution 18 is maintained at the target temperature, a temperature in the space 11A can also be maintained at substantially the same temperature. As described above, the composition ratio of the mixed gas 31 in the space 11A is maintained at the target value outside the preset explosion range. The target value also includes the water vapor concentration of the mixed gas 31. A temperature of the mixed gas 31 can be made to be 40°C or higher by controlling the temperature of the culture solution 18 to 40°C or higher. As a result, the water vapor concentration of the mixed gas 31 can also be adjusted to 7% or more. The processor 17, the thermometer 46, and the temperature adjuster 47 are examples of a "temperature control mechanism" according to the technology of the present disclosure.

In addition, the processor 17 controls a pressure of the mixed gas 31 in the space 11A based on the pressure PS measured by the pressure gauge 48. By setting the pressure PS of the mixed gas 31 to a target pressure, solubility of the raw material gas 26 in the culture solution 18 is improved. In this way, the processor 17 controls the pressure of the mixed gas 31 to ensure appropriate solubility in the culture solution 18. As a result, culture efficiency of the hydrogen-oxidizing bacteria 21 is improved. An adjustment target of the pressure PS of the mixed gas 31 is at least one of the supply amount of the raw material gas 26, the discharge amount of the mixed gas 31, or the supply amount of the adjustment gas 33. The processor 17 controls driving of each pump 22 and the pump 37 to adjust the supply amount or the discharge amount of the gas. The processor 17, the raw material gas tank 12, the adjustment gas tank 14, the pumps 22 on the supply paths 12A and 14A, and the pump 37 on the discharge path 36 are examples of a "pressure control mechanism" according to the technology of the present disclosure.

The processor 17 is configured of, for example, a central processing unit (CPU) and a random access memory (RAM), and controls each unit of the culture device 10 as described above. In addition, the memory 17A is a non-volatile memory in which various types of setting information are stored, such as a flash memory.

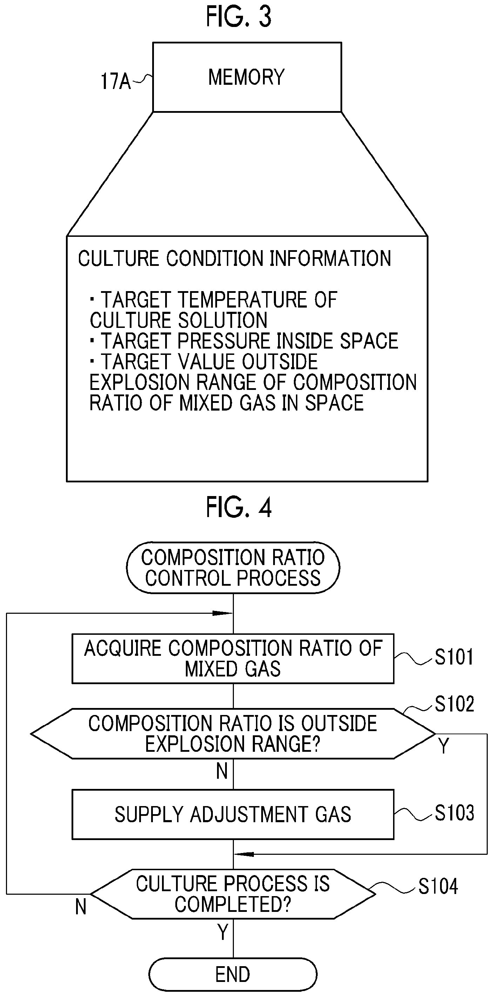

As shown in Fig. 3, the memory 17A stores, as culture condition information, information such as the target temperature of the culture solution 18, the target pressure inside the space 11A, and the target value outside the explosion range of the composition ratio of the mixed gas 31 in the space 11A. The processor 17 performs various types of control by referring to such culture condition information.

Hereinafter, an operation of the above-mentioned configuration will be described with reference to a flowchart shown in Fig. 4. Fig. 4 shows a procedure of a composition ratio control process. For example, the processor 17 drives the temperature adjuster 47 in a state in which the culture solution 18 containing the hydrogen-oxidizing bacteria 21 is accommodated in the culture tank 11, to heat the temperature of the culture solution 18 to the target temperature. The processor 17 starts a culture process by starting the supply of the raw material gas 26 after the culture solution 18 reaches the target temperature. Even after the start of the culture process, the processor 17 controls the temperature of the culture solution 18 to the target temperature through the temperature adjuster 47. In the culture process, the hydrogen-oxidizing bacteria 21 proliferate by taking in the raw material gas 26 dissolved in the culture solution 18, and further produce the organic substance 29. The hydrogen-oxidizing bacteria 21 efficiently produce the organic substance 29 by modifying the gene. A timing at which the hydrogen-oxidizing bacteria 21 produce the organic substance 29 is during or after the proliferation. A part of the raw material gas 26 that is not dissolved in the culture solution 18 is released into the space 11A above the liquid surface 18A of the culture solution 18, and becomes the mixed gas 31.

In step S101 of the composition ratio control process, the processor 17 acquires the measured values of the concentration of each component of the mixed gas 31 and the total flow rate of the mixed gas 31 from the composition ratio measurement unit 16. Then, the processor 17 calculates the oxygen concentration of the mixed gas 31 based on the acquired measurement values, and acquires the calculated oxygen concentration as the composition ratio of the mixed gas 31.

In step S102, the processor 17 determines whether or not the composition ratio of the mixed gas 31 is outside the explosion range. As described above, in the present example, the target value outside the explosion range is set to an oxygen concentration of 5% or more and 9% or less and a water vapor concentration of 7% or more. In addition, in the present example, the target temperature of the culture solution 18 is set to 40°C or higher. Therefore, since a saturated water vapor amount at 40°C is approximately 7% in terms of the water vapor concentration, in a case where the target temperature of the culture solution 18 is set to 40°C or higher, the water vapor concentration of the mixed gas 31 can be adjusted to 7% or more. The processor 17 compares the oxygen concentration derived from the measured values of the composition ratio measurement unit 16 with the target value. In a case where the oxygen concentration is within a range of the target value, it is determined that the composition ratio is outside the explosion range (Y in step S102), and the process proceeds to step S104.

On the other hand, in a case where the oxygen concentration exceeds 9% which is an upper limit value of the target value, the processor 17 determines that the composition ratio of the mixed gas 31 is in the explosion range (N in step S102), and the process proceeds to step S103. In step S103, the processor 17 drives the pump 22 of the supply path 14A to supply the adjustment gas 33 to the space 11A. As a result, the oxygen concentration of the mixed gas 31 decreases, and the composition ratio of the mixed gas 31 can be returned to the outside of the explosion range.

The processor 17 repeats such a composition ratio control process until the culture process is completed (Y in step S104). As a result, the composition ratio of the mixed gas 31 in the space 11A can be maintained at the target value outside the explosion range.

As described above, the culture device 10 according to the technology of the present disclosure comprises the composition ratio measurement unit 16 that measures the composition ratio of the mixed gas 31 in the space 11A present above the liquid surface 18A of the culture solution 18 in the culture tank 11, and the composition ratio control mechanism that supplies the adjustment gas 33 into the space 11A according to the measured composition ratio of the mixed gas 31 to maintain the composition ratio of the mixed gas 31 in the space 11A outside the preset explosion range. Therefore, in a case of culturing microorganisms (for example, the hydrogen-oxidizing bacteria 21) that use the raw material gas 26 containing a flammable gas (for example, hydrogen) for the proliferation or for the production of the organic substance, the culture efficiency and safety can be improved as compared with the related art.

That is, since the culture device 10 of the present disclosure maintains the composition ratio of the mixed gas 31 in the space 11A at the target value outside the preset explosion range, the safety is improved as compared with a case where the dissolved oxygen concentration in the culture solution 18 is controlled as in JP2564008B.

In addition, in the culture device 10 according to the present disclosure, since the culture solution 18 does not circulate in a hydrogen tank and an oxygen tank that are provided separately from the culture tank 11 unlike JP1976-038480A (JP-S51-038480A), the composition ratio of the raw material gas 26 in the culture solution 18 can always be maintained at an appropriate value. Therefore, the culture efficiency is improved as compared with JP1976-038480A (JP-S51-038480A). In addition, since the culture device 10 of the present disclosure does not perform control of reducing the supply amount of the raw material gas 26 such as an oxygen supply amount for the purpose of maintaining the composition ratio of the mixed gas 31 outside the explosion range unlike JP2564008B, the raw material gas 26 necessary for the proliferation of the microorganisms or the production of the organic substance is not deficient. Therefore, the culture device 10 of the present disclosure improves the culture efficiency of the microorganism as compared with JP2564008B.

In addition, in the present embodiment, the adjustment gas 33 is an inert gas, which provides a high explosion prevention effect and is safe.

In addition, in the present example, the target value outside the explosion range of the mixed gas 31 is an oxygen concentration of 5% or more and 9% or less and a water vapor concentration of 7% or more. By setting the water vapor concentration to 7% or more, the oxygen concentration can be set to be higher than that in a case where the water vapor concentration is less than 7%. Since oxygen is one of the components of the raw material gas 26, the oxygen concentration is set to be high, whereby the oxygen as the raw material gas 26 can be supplied in a larger amount. Therefore, good culture efficiency can be ensured. In addition, even in a state in which the oxygen concentration is relatively high, since the composition ratio of the mixed gas 31 can be maintained at the target value outside the explosion range, an increase in the supply amount of the adjustment gas 33 for lowering the oxygen concentration is suppressed. Accordingly, wasteful consumption of the adjustment gas 33 can be suppressed.

In addition, the target temperature of the culture solution 18 is set to 40°C or higher. In a case where the temperature of the culture solution 18 is 40°C or higher, it is possible to adjust the water vapor concentration of the mixed gas 31 to 7% or more. Therefore, it is possible to improve the culture efficiency and the safety.

As described above, the target value of the composition ratio of the mixed gas 31 outside the explosion range may be set to an oxygen concentration of less than 5%. In this way, since the explosion prevention effect can be expected regardless of the water vapor concentration, high safety can be ensured. In addition, the hydrogen concentration may be set to less than 4% instead of setting the oxygen concentration to less than 5%. Even in this case, the explosion prevention effect can be expected regardless of the water vapor concentration, and high safety can be ensured. In a case of culturing a microorganism of which a consumption amount of hydrogen is relatively large, such as the hydrogen-oxidizing bacteria 21, it is preferable to set a target value of the oxygen concentration as the target value outside the explosion range and control the oxygen concentration. This is because, in a case where the consumption amount of hydrogen of the microorganism is large, it is necessary to increase the hydrogen concentration of the raw material gas 26 in order to increase a supply amount of hydrogen, and as a result, the hydrogen concentration of the mixed gas 31 also tends to increase. This is because, in that case, it is difficult to control the hydrogen concentration of the mixed gas 31 to a low concentration, and it is easier to control the oxygen concentration to a low concentration than the hydrogen concentration.

In addition, in the present example, the foam layer 18B is formed on the liquid surface 18A of the culture solution 18 by the stirring rod 41 shown as an example of the foam layer forming portion. Therefore, since the heat capacity in the space 11A increases, the explosion prevention effect is also improved.

In addition, in the present example, the circulation path 39 for reusing the mixed gas 31 in the space 11A as the raw material gas 26 is provided. The mixed gas 31 includes the raw material gas 26. Therefore, it is possible to suppress wasteful consumption of the raw material gas 26 as compared with a case where the mixed gas 31 is not reused. In addition, in the present example, since the adjustment gas 33 is one of the components of the raw material gas 26, the adjustment gas 33 is also reused as a raw material. Therefore, the adjustment gas 33 can be effectively used. In a case where a component other than the components of the raw material gas 26 is used as the adjustment gas 33, only the components obtained by excluding the adjustment gas 33 from the mixed gas 31 may be reused as the raw material gas 26.

In addition, in a case where a component other than the components of the raw material gas 26 is used as the adjustment gas 33, the adjustment gas 33 that has been once exhausted from the space 11A may be reused as the adjustment gas 33.

In addition, in the present example, the adjustment gas 33 is carbon dioxide. Since carbon dioxide is one of the components of the raw material gas 26 in the present example, in a case where a part of the carbon dioxide is dissolved in the culture solution 18 by supplying the adjustment gas 33, the raw material gas 26 can be replenished, and an effect can be expected from the viewpoint of culture efficiency. Further, in a case where the mixed gas 31 is reused as in the present example, the use of carbon dioxide, which is one of the components of the raw material gas 26, as the adjustment gas 33 has the following advantages. That is, as described above, since the adjustment gas 33 is one of the components contained in the raw material gas 26, the raw material gas 26 is not diluted by other components that are not used as the raw material.

In addition, in the present example, the microorganisms are the hydrogen-oxidizing bacteria 21. Since the hydrogen-oxidizing bacteria 21 use carbon dioxide as the raw material gas 26, there is an advantage in suppressing greenhouse gases.

In addition, in the present example, a pressure measurement unit (for example, the pressure gauge 48) that measures the pressure PS in the space 11A, and a pressure control mechanism that controls the pressure by adjusting at least one of the supply amount of the raw material gas 26, the discharge amount of the mixed gas 31, or the supply amount of the adjustment gas 33 based on the measured pressure PS are provided. By controlling the pressure inside the space 11Ato the target pressure, it is possible to ensure appropriate solubility of the raw material gas 26 in the culture solution 18.

### [Second Embodiment]

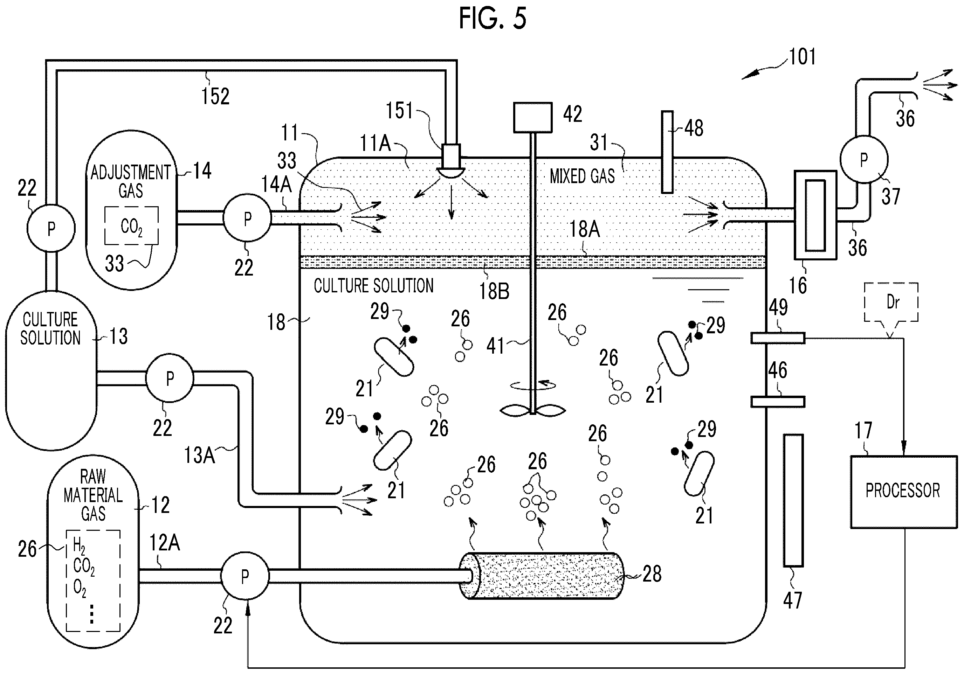

Fig. 5 shows a culture device 101 according to a second embodiment. In the second embodiment, the same parts as those in the first embodiment will not be described, and differences will be mainly described. For convenience, a part of a configuration shown in Fig. 5 is also omitted. The second embodiment has two main differences from the first embodiment as follows. First, the culture device 101 comprises a scattering mechanism 151 that scatters a liquid containing water into the space 11A. Secondly, the culture device 101 comprises a dissolved gas concentration measurement unit 49 that measures a dissolved gas concentration Dr of the raw material gas 26 dissolved in the culture solution 18, and a raw material gas control mechanism that controls the supply amount of the raw material gas 26 according to the dissolved gas concentration Dr.

The scattering mechanism 151 is, for example, a sprinkler type or spray type liquid scattering mechanism, and is provided on a ceiling portion of the space 11A. The liquid to be scattered may be water, but in the present example, the liquid is the culture solution 18. By scattering the liquid containing water into the space 11A, the explosion prevention effect is enhanced, and the safety is improved. In addition, by scattering the culture solution 18, a decrease in the concentration of the raw material gas 26 in the culture solution 18 can be suppressed. In this manner, a decrease in the culture efficiency is suppressed. The liquid to be scattered does not have to be the culture solution 18 itself, and may be an aqueous solution containing at least a part of the components of the culture solution 18.

The culture solution 18 is supplied to the scattering mechanism 151 from the culture solution tank 13 via a supply path 152. The processor 17 controls a scattering amount of the culture solution 18 scattered by the scattering mechanism 151 through the pump 22 of the supply path 152.

As shown in Fig. 6, more specifically, the dissolved gas concentration measurement unit 49 measures the concentration of each component (hydrogen, oxygen, and carbon dioxide) contained in the raw material gas 26 dissolved in the culture solution 18. As an example, the dissolved gas concentration measurement unit 49 is composed of a dissolved hydrogen meter (for example, model number KM2100DH manufactured by Kyoei Denshi Kenkyusho) that measures an amount of dissolved hydrogen, a dissolved oxygen meter (for example, model number InPro6850i manufactured by Mettle Toledo) that measures the dissolved oxygen concentration, and a dissolved carbon dioxide meter (for example, model number InPro5000i manufactured by Mettle Toledo) that measures dissolved carbon dioxide. The dissolved gas concentration measurement unit 49 outputs measured values to the processor 17.

The raw material gas tank 12 accommodates each component (hydrogen, oxygen, and carbon dioxide) of the raw material gas 26. In addition, the supply path 12A and the pump 22 are provided for each component. The raw material gas tank 12, the supply path 12A, and the pump 22 are examples of a "raw material gas control mechanism" according to the technology of the present disclosure.

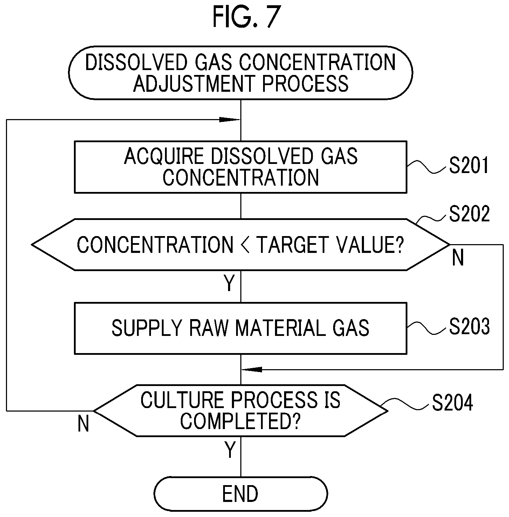

As shown in Fig. 7, in step S201 of a dissolved gas concentration adjustment process, the processor 17 acquires a measured value of the dissolved gas concentration for each component from the dissolved gas concentration measurement unit 49. In step S202, the processor 17 compares the dissolved gas concentration with the target value for each component, and determines whether or not there is a component that is deficient in the raw material gas 26. In a case where there is a deficient component (Y in step S202), in step S203, the pump 22 on the supply path 12A is driven to supply the deficient component in the raw material gas 26 to the culture tank 11. Such a process is repeated until the culture process is completed (step S204). By such a dissolved gas concentration adjustment process, the dissolved gas concentration of the raw material gas 26 in the culture solution 18 can be maintained at an appropriate value. The culture efficiency of the microorganisms can be improved, as compared with a case where such a process is not performed.

In the present example, although an example in which the dissolved gas concentration is measured for each component of the raw material gas 26 and replenishment is performed for each deficient component has been described, a total dissolved gas concentration of the raw material gas 26 may be measured instead of each component, and the raw material gas 26 may be replenished in total. Even in this case, the shortage of the raw material gas 26 can be suppressed. Of course, as shown in Figs. 6 and 7, it is preferable to replenish the raw material gas 26 for each component since the composition ratio of the raw material gas 26 can be maintained within an appropriate range.

### [Third Embodiment]

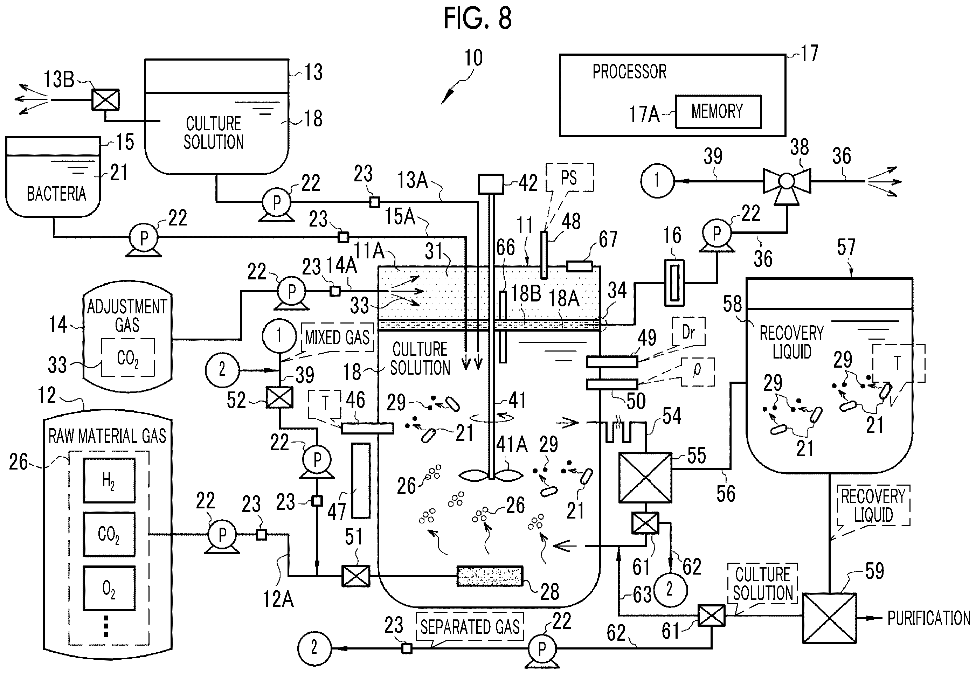

The culture device 10 according to a third embodiment shown in Figs. 8 to 15 is the same culture device as the above-described embodiments, and various additional configurations are added thereto. In the following description, parts common to the above-described embodiments are basically denoted by the same reference numerals. In the third embodiment, some of the description overlaps with the above-mentioned embodiments. However, since the overlapping parts are useful in showing a relationship between the additional configuration added in the third embodiment and a basic configuration, some overlapping parts will be described. It goes without saying that at least a part of the third embodiment and each of the above-described embodiments can be appropriately combined within a range that does not cause contradiction.

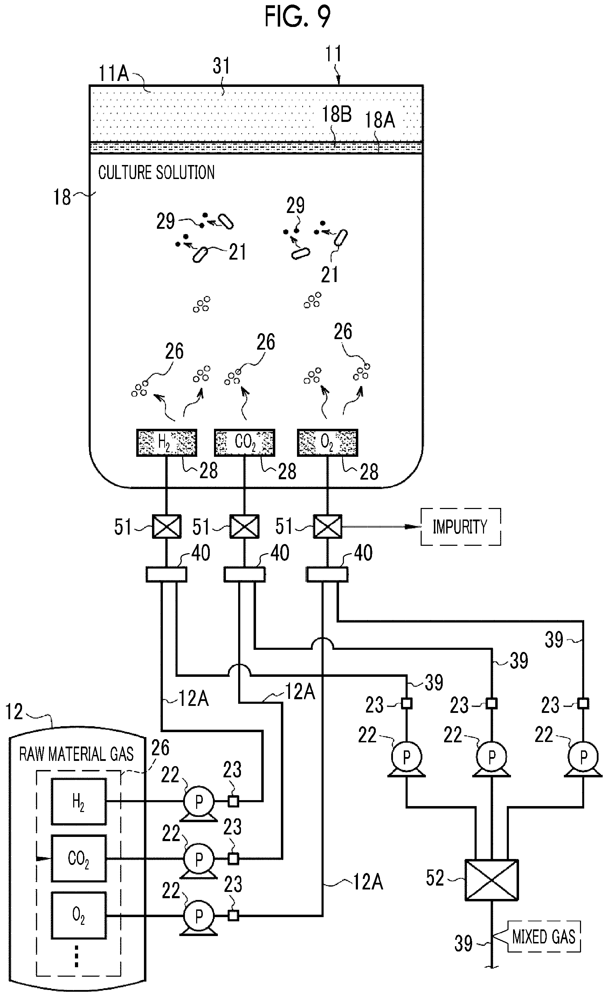

As shown in Fig. 9, the culture device 10 according to the third embodiment cultures the hydrogen-oxidizing bacteria, which are microorganisms, as an example of a culture target, as in each of the above-described embodiments. The raw material gas 25 and the like are the same.

As shown in Fig. 9, the culture device 10 comprises a culture tank 11, a raw material gas tank 12, a culture solution tank 13, an adjustment gas tank 14, a culture target tank 15, and a processor 17. The culture tank 11 accommodates a culture solution 18. The hydrogen-oxidizing bacteria 21 and the culture solution 18 are the same as those in the above-described embodiment. In addition to or instead of the raw material gas tank 12, a gas generation device that generates the raw material gas 26 may be provided.

Each of supply paths 12A, 13A, 14A, and 15A of each of the raw material gas tank 12, the culture solution tank 13, the adjustment gas tank 14, and the culture target tank 15 is connected to the culture tank 11. Each of the supply paths 12A, 13A, 14A, and 15A is composed of a pipe, a valve, and the like. In addition, a flowmeter 23 that measures a flow rate of a fluid flowing through the pump 22 and each supply path is disposed in each of the supply paths 12A, 13A, 14A, and 15A.

The culture solution 18 is accommodated in the culture solution tank 13. The culture solution 18 is supplied from the culture solution tank 13 to the culture tank 11 through the supply path 13A by driving the pump 22 on the supply path 13A. A supply timing and a supply amount of the culture solution 18 are controlled by the processor 17 that controls the driving of the pump 22.

### (Pretreatment for Culture Solution in Culture Solution Tank)

In addition, as an example, a deaerator 13B is provided in the culture solution tank 13. In a case where the culture solution 18 is replenished from the culture solution tank 13 to the culture tank 11, the deaerator 13B removes an unnecessary gas dissolved in the culture solution 18. The unnecessary gas is, for example, a gas that adversely affects culture, such as a decrease in the culture efficiency, such as air and nitrogen.

The culture solution tank 13 and the raw material gas tank 12 may be connected by a pipe or the like so that the raw material gas can be replenished to the culture solution 18 before being supplied to the culture tank 11. By replenishing the raw material gas in advance to the culture solution 18 before being supplied to the culture tank 11, the dissolved gas concentration of the raw material gas in the culture solution 18 supplied to the culture tank 11 can be improved. In this manner, the culture efficiency may be improved. In this case, a dissolved gas concentration measurement unit (similar to a dissolved gas concentration measurement unit 49 described later) that measures the dissolved gas concentration of the raw material gas of the culture solution 18 may be provided in the culture solution tank 13, and a replenishment amount of the raw material gas may be controlled according to the measured dissolved gas concentration.

The culture target tank 15 accommodates the hydrogen-oxidizing bacteria 21 which are an example of the culture target. The hydrogen-oxidizing bacteria 21 are supplied from the culture target tank 15 to the culture tank 11 through the supply path 15A by driving the pump 22 on the supply path 15A. A supply timing and a supply amount of the hydrogen-oxidizing bacteria 21 are controlled by the processor 17 that controls the driving of the pump 22.

A raw material gas 26 is accommodated in the raw material gas tank 12. As described above, the raw material gas 26 is, for example, a gas containing each component such as hydrogen (H₂), oxygen (O₂), and carbon dioxide (CO₂). The raw material gas 26 is supplied from the raw material gas tank 12 to the culture tank 11 through the supply path 12A by driving the pump 22 on the supply path 12A. A supply timing and a supply amount of the raw material gas 26 are controlled by the processor 17 that controls the driving of the pump 22.

A downstream end of the supply path 12Ais disposed in the culture tank 11, and a sparger 28 is provided at the downstream end. The sparger 28 is provided for dispersing and supplying the raw material gas 26 into the culture solution 18. As the sparger 28, for example, a ventilation pipe formed of a porous material is used. The sparger 28 is an example of a bubble generator that generates the raw material gas 26 as bubbles in the culture solution 18. The bubble generator includes various types such as a microhole type like the sparger 28, a swirling flow liquid type that generates bubbles by a high-speed swirling flow, an ejector type (also referred to as a Venturi type) that generates bubbles by a rapid pressure change in a gas-liquid flow path, and a static mixer type that generates bubbles by a shearing force of an obstacle in a gas-liquid flow path. As the bubble generator, a type other than the sparger 28 may be used.

A part of the raw material gas 26 supplied into the culture solution 18 is dissolved in the culture solution 18. The dissolved raw material gas 26 is taken in by the hydrogen-oxidizing bacteria 21 and is consumed for the proliferation of the hydrogen-oxidizing bacteria 21 or the production of the organic substance 29. The organic substance 29 is, for example, alcohol or an amino acid. The raw material gas 26 that is not dissolved in the culture solution 18 is released into a space 11A present above a liquid surface 18A of the culture solution 18. The space 11A is filled with a mixed gas 31 which is the raw material gas 26 released without being dissolved in the culture solution 18. The raw material gas tank 12 and the pump 22 on the supply path 12A are examples of a "raw material gas supply unit".

An adjustment gas 33 is accommodated in the adjustment gas tank 14. The adjustment gas 33 is supplied from the adjustment gas tank 14 to the space 11A of the culture tank 11 through the supply path 14A by driving the pump 22 on the supply path 14A. A supply timing and a supply amount of the adjustment gas 33 are controlled by the processor 17 that controls the driving of the pump 22. The mixed gas 31 in the space 11A includes hydrogen and oxygen, which are components of the raw material gas 26. The hydrogen is a flammable gas. As will be described later, the adjustment gas 33 is used to maintain the composition ratio of the mixed gas 31 in the space 11A at a target value outside a preset explosion range.

In addition, the mixed gas 31 includes the components of the raw material gas 26. Therefore, a part of the mixed gas 31 can be returned to the culture tank 11 via a gas circulation path 39 and reused as the raw material gas 26. The mixed gas 31 to be reused as the raw material gas 26 is also supplied to the culture tank 11 via the sparger 28. The mixed gas 31 that is not reused is discharged. Although not shown, in a case where the mixed gas 31 that is not reused is discharged, the mixed gas 31 is diluted with an inert gas such as carbon dioxide and then released into the atmosphere.

A discharge path 36 for extracting the mixed gas 31 is connected to the culture tank 11, and a pump 22 and a three-way valve 38 are provided on the discharge path 36. The pump 22 generates a pressure for discharging and circulating the mixed gas 31. The three-way valve 38 switches between a state in which the discharge path 36 communicates with the gas circulation path 39 and a state in which the communication between the discharge path 36 and the gas circulation path 39 is closed. The three-way valve 38 switches whether the mixed gas 31 is sent to the gas circulation path 39 or is discharged to the outside. In addition, the discharge path 36 is provided with a composition ratio measurement unit 16 that measures the composition ratio of the mixed gas 31. The composition ratio measurement unit 16 is used for controlling the composition ratio of the mixed gas 31 described later.

### (Position of Mixed Gas Extraction Port)

In addition, a mixed gas extraction port 34 for extracting the mixed gas 31 from the culture tank 11 is disposed at a position where the foam layer 18B formed on the liquid surface 18A of the culture solution 18 in the culture tank 11 can be taken in. The foam layer 18B functions as a liquid film that covers the liquid surface 18A and increases the heat capacity of the space 11A to contribute to the suppression of the explosion. However, on the other hand, there may be an adverse effect on various sensors disposed in the culture tank 11, as well as a concern that the hydrogen-oxidizing bacteria 21 and the like may be damaged. Therefore, by disposing the mixed gas extraction port 34 at a position where the foam layer 18B can be taken in, it is possible to recover a part of the foam layer 18B in a case where the mixed gas 31 is extracted, and it is possible to reduce the adverse effect caused by the foam layer 18B.

### (Supply of Each Component of Raw Material Gas)

In addition, in Fig. 8, for convenience of simplifying the drawing, the supply path 12A of the raw material gas 26 and the gas circulation path 39 of the mixed gas 31 are shown as one. However, in reality, as shown in Fig. 9, the supply path 12A and the gas circulation path 39 are separated for each component such as hydrogen, oxygen, and carbon dioxide, and a plurality of components can be individually supplied. A pump 22 and a flowmeter 23 are provided in each supply path 12A and gas circulation path 39 for each component. In addition, the sparger 28 is also provided for each component such as hydrogen (H₂), oxygen (O₂), and carbon dioxide (CO₂). Each supply path 12A and gas circulation path 39 for each component are connected to each sparger 28 corresponding to each component.

### (Removal of Impurities from Raw Material Gas)

A connecting member 40 connects the supply path 12A and the gas circulation path 39 to each other on an upstream side of the sparger 28 for each component. As a result, the supply path 12A and the gas circulation path 39 are integrated into one and are connected to the sparger 28. An impurity removal mechanism 51 is provided between each connecting member 40 and each sparger 28. The impurity removal mechanism 51 removes impurities from the raw material gas 26 supplied to the culture tank 11. The impurities include sulfur oxide or nitrogen oxide (NO, NO₂, and the like). The impurity removal mechanism 51 is a mechanism that uses any of a membrane separation method, a cryogenic separation method, a physical adsorption method, or a chemical absorption method, as a method of separating impurities from the raw material gas 26.

In Fig. 8, the impurities accumulate in the culture tank 11 in a case where the culture process is continued. As will be described later, a part of the culture solution 18 in the culture tank 11 is extracted once and returned to the culture tank 11 to be reused. In a case where the culture solution 18 circulates, the raw material gas 26 is separated from the culture solution 18, and the raw material gas 26 in the culture solution 18 is also returned to the culture tank 11 via a gas reuse path 62 to be reused. In addition, a part of the mixed gas 31 is also returned to the culture tank 11 via the gas circulation path 39 to be reused. The gas reuse path 62 and the gas circulation path 39 are connected to an upstream side of the impurity removal mechanism 51. The mixed gas 31 to be reused and a separated gas separated from the culture solution 18 are resupplied to the culture tank 11 after the impurities are removed by the impurity removal mechanism 51. Since the impurities are removed while circulating the raw material gas 26 in this way, accumulation of the impurities in the culture tank 11 can be suppressed.

### (Separation of Components Contained in Mixed Gas)

In addition, since the components such as hydrogen, oxygen, and carbon dioxide are mixed in the mixed gas 31, the gas circulation path 39 is provided with a component separation mechanism 52 that separates each component contained in the mixed gas 31. Each component contained in the mixed gas 31 is separated by the component separation mechanism 52, and each separated component is supplied to each gas circulation path 39 provided for each component. The component separation mechanism 52 is a mechanism that uses any of a membrane separation method, a cryogenic separation method, a physical adsorption method, or a chemical absorption method, as a method of separating each component from the mixed gas 31. The component separation mechanism 52 is an example of a "first component separation mechanism" according to the technology of the present disclosure.

As described above, in a case where the raw material gas 26 is supplied to the culture tank 11, there are advantages in terms of culture efficiency and safety by individually supplying each component such as hydrogen (H₂), oxygen (O₂), and carbon dioxide (CO₂). The advantage of the culture efficiency is that the raw material gas 26 supplied to the culture tank 11 is easily adjusted to an appropriate composition from the viewpoint of the culture efficiency. In addition, the advantages of the safety are as follows. That is, three elements of explosion are a flammable gas, oxygen, and an ignition source, but before the raw material gas 26 is supplied to the culture tank 11, hydrogen, which is a flammable gas, and oxygen among the three elements of explosion can be separated and handled, and a risk of explosion can be further reduced.

The component separation mechanism 52 may also separate components such as nitrogen and methane as the components contained in the mixed gas 31, in addition to hydrogen, oxygen, and carbon dioxide. Since nitrogen, methane, and the like have an action of reducing the solubility of the raw material gas 26 in the culture solution 18, in a case where the mixed gas 31 is reused, the solubility of the raw material gas 26 can be improved by separating these gases.

### (Circulation, Recovery, and Reuse of Culture Solution)

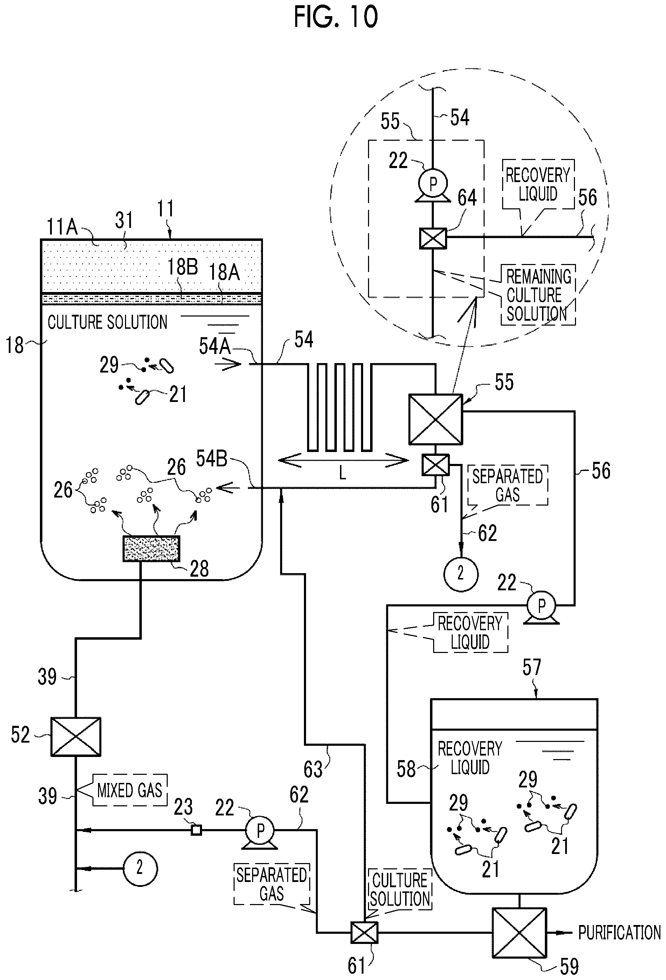

As shown in Fig. 10 in addition to Fig. 8, a culture solution circulation path 54 is connected to the culture tank 11, and a culture solution recovery mechanism 55 is provided in the culture solution circulation path 54. The culture solution circulation path 54 extracts a part of the culture solution 18 from the culture tank 11. The culture solution recovery mechanism 55 recovers the culture solution 18 including the organic substance 29 from the culture solution 18 extracted from the culture tank 11 through the culture solution circulation path 54. The culture solution circulation path 54 returns the culture solution 18 containing the hydrogen-oxidizing bacteria 21, which is a remaining culture solution 18 after being recovered by the culture solution recovery mechanism 55, to the culture tank 11. In a case where the proliferated hydrogen-oxidizing bacteria 21 are purified, the proliferated hydrogen-oxidizing bacteria 21 are recovered, for example, by a method different from the method for the organic substance 29.

### (Separation of Dissolved Gas Contained in Culture Solution)

In the culture solution circulation path 54, a gas separation mechanism 61 is provided on a downstream side of the culture solution recovery mechanism 55. The gas separation mechanism 61 separates a gas containing the components of the raw material gas 26 contained in the culture solution 18 from the culture solution 18 as a separated gas before returning the culture solution 18 to the culture tank 11. Then, the culture solution 18 from which the separated gas has been separated is returned to the culture tank 11. The gas separation mechanism 61 is a separation mechanism that uses any of a membrane separation method, a cryogenic separation method, a physical adsorption method, or a chemical absorption method. A hydrogen storage alloy may be used for the separation of hydrogen.

### (Reuse of Raw Material Gas from Culture Solution)

A part of the separated gas separated by the gas separation mechanism 61 is returned to the culture tank 11 via the gas reuse path 62 and the gas circulation path 39 of the mixed gas 31. Then, the part of the separated gas is reused as the raw material gas 26. Therefore, use efficiency of the raw material gas 26 is improved.

### (Explosion Suppression in Case of Reusing Raw Material Gas from Culture Solution)

In addition, since the separated gas is returned to the culture tank 11 via the gas circulation path 39, the separated gas is separated into respective components such as hydrogen, oxygen, and carbon dioxide by the component separation mechanism 52 as described above. However, the separated gas may be separated into respective components such as hydrogen, oxygen, and carbon dioxide in the gas reuse path 62 instead of being separated for each component in the component separation mechanism 52. Each of these separated components is reused via the gas circulation path 39 provided for each component. The separation of the separated gas in the gas reuse path 62 into each component may be performed, for example, by the gas separation mechanism 61 that separates the gas from the culture solution 18.

In this way, in a case where the separated gas is reused, hydrogen, which is a flammable gas, and oxygen can be separated, so that the safety in the gas reuse path 62 is improved. As described above, from the viewpoint of safety, it is preferable to separate hydrogen, which is a flammable gas, from the culture solution 18 extracted from the culture tank 11 as upstream as possible. As for the order of separating the components, it is preferable to separate hydrogen first and then separate oxygen and carbon dioxide. Such a gas separation mechanism 61 has a function of suppressing explosion, and is therefore an example of an "explosion suppression mechanism" according to the technology of the present disclosure.

In addition, in a case where the respective components of the separated gas are separated in the gas separation mechanism 61 in this way, the carbon dioxide can be reused as the raw material gas 26 and can also be returned to the adjustment gas tank 14 to be reused as the adjustment gas 33. The gas separation mechanism 61 is also an example of a "second component separation mechanism" according to the technology of the present disclosure in a case of separating the separated gas into each component. Also in the gas separation mechanism 61, as in the component separation mechanism 52, components that reduce the solubility of the raw material gas 26, such as nitrogen and methane, may also be separated in addition to hydrogen, oxygen, and carbon dioxide.

### (Separation of Specific Component from Culture Solution)

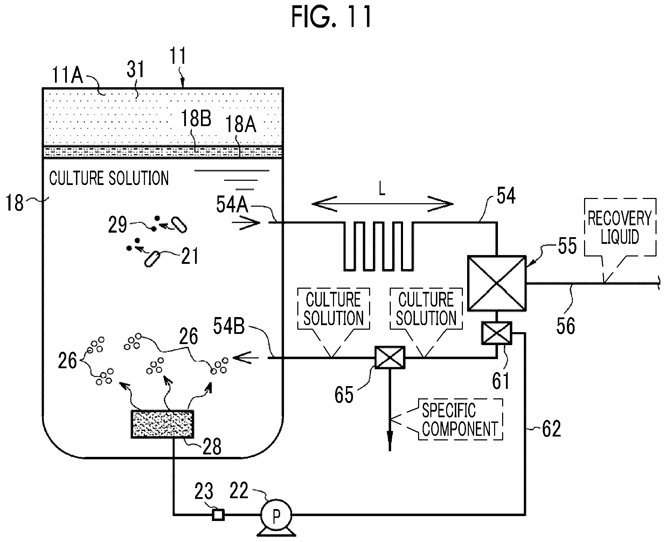

In addition, as shown in Fig. 11, by using a separation mechanism 65 similar to the gas separation mechanism 61 and the component separation mechanism 52, in addition to separating the gas from the culture solution 18, a specific component contained in the culture solution 18 may be separated. Examples of the specific component include ammonia and an ammonium compound. The ammonia and the ammonium compound serve as a nutrient source for the hydrogen-oxidizing bacteria 21. By separating these, it is possible to reuse the separated components while adjusting the supply amount.

In this way, by separating various components contained in the mixed gas 31 and the culture solution 18, it is possible to remove unnecessary components and reuse those that can be reused, and it is possible to improve the culture efficiency and the use efficiency of the raw material gas 26.

In Fig. 10, the culture solution recovery mechanism 55 is connected to a recovery liquid tank 57 that stores a recovery liquid 58 which is the recovered culture solution 18, via a recovery path 56. The recovery liquid 58 is transferred from the culture solution recovery mechanism 55 to the recovery liquid tank 57 through the recovery path 56. A pump 22 for transferring the recovery liquid 58 is provided in the recovery path 56.

A part of the recovery liquid 58 accommodated in the recovery liquid tank 57 is transferred to a purification device (not shown). In the purification device, the organic substance 29 produced by culturing the hydrogen-oxidizing bacteria 21 is extracted from the recovery liquid 58.

### (Process of Reducing Dissolved Flammable Gas Concentration in Recovery Liquid)

The culture device 10 comprises a concentration reduction mechanism that reduces a dissolved hydrogen concentration in the recovery liquid 58 to be lower than a dissolved hydrogen concentration in the culture solution 18 in the culture tank 11. The concentration reduction mechanism is configured, for example, by extending a total length L of a section from the culture tank 11 to the culture solution recovery mechanism 55 in the culture solution circulation path 54 to a length required for reducing the concentration. Specifically, the total length L of the section has a length required to reduce the dissolved hydrogen concentration in the recovery liquid 58 to 1/10 or less of the dissolved hydrogen concentration in the culture solution 18 in the culture tank 11. The culture solution 18 extracted from the culture tank 11 contains the hydrogen-oxidizing bacteria 21 that consume hydrogen, and the hydrogen-oxidizing bacteria 21 also consume hydrogen in the culture solution circulation path 54. As the culture solution circulation path 54 is longer, a time for which the hydrogen is consumed by the hydrogen-oxidizing bacteria 21 is ensured. In this manner, use efficiency of hydrogen is improved. In addition, in a case where hydrogen is consumed in a section of the culture solution circulation path 54 up to the culture solution recovery mechanism 55, the dissolved hydrogen concentration of the recovery liquid 58 recovered by the culture solution recovery mechanism 55 is reduced. As a result, the hydrogen released from the recovery liquid 58 is also reduced, so that the risk of explosion in the recovery liquid tank 57 is also reduced, and the safety is ensured. The culture solution circulation path 54 of which the total length L of the above-described section has a length required for reducing the concentration is an example of a "concentration reduction mechanism" according to the technology of the present disclosure.

In addition, instead of extending the total length L of the section of the culture solution circulation path 54, a flow rate of the culture solution 18 flowing through the section may be decreased. As a result, since a time until the culture solution 18 extracted from the culture tank 11 is transferred to the culture solution recovery mechanism 55 is extended, a time for which the hydrogen is consumed in the extracted culture solution 18 can be extended. As a result, the same effect as in a case where the total length L of the section is extended can be obtained. The flow rate of the culture solution 18 can be adjusted, for example, by controlling a suction amount of the pump 22 per unit time. The suction amount of the pump 22 is controlled by the processor 17. A flow rate adjustment mechanism configured by the processor 17 and the pump 22 is also an example of a "concentration reduction mechanism" according to the technology of the present disclosure.

### (Downstream Side of Recovery Liquid Tank 57)

On a downstream side of the recovery liquid tank 57, a purification target separation mechanism 59 is provided to separate a part of the recovery liquid 58, which contains the organic substance 29, from the recovery liquid 58 as a purification target and send the separated purification target to the purification device. The remaining recovery liquid 58 other than the recovery liquid 58 separated as the purification target by the purification target separation mechanism 59 is returned to the culture tank 11 as the culture solution 18 via a liquid reuse path 63 and the culture solution circulation path 54 and reused.

As shown in an enlarged view of a part of Fig. 10, the culture solution recovery mechanism 55 comprises the pump 22 and a liquid separation mechanism 64. The pump 22 generates a circulation pressure for circulating the culture solution 18. Similar to the purification target separation mechanism 59, the liquid separation mechanism 64 separates a part of the culture solution 18, which includes the organic substance 29, from the culture solution 18 extracted from the culture tank 11, and sends the separated culture solution 18 to the recovery path 56 as the recovery liquid 58. The remaining culture solution 18 is sent to the gas separation mechanism 61.

### (Position of Extraction Port of Culture Solution in Circulation Path of Culture Solution)

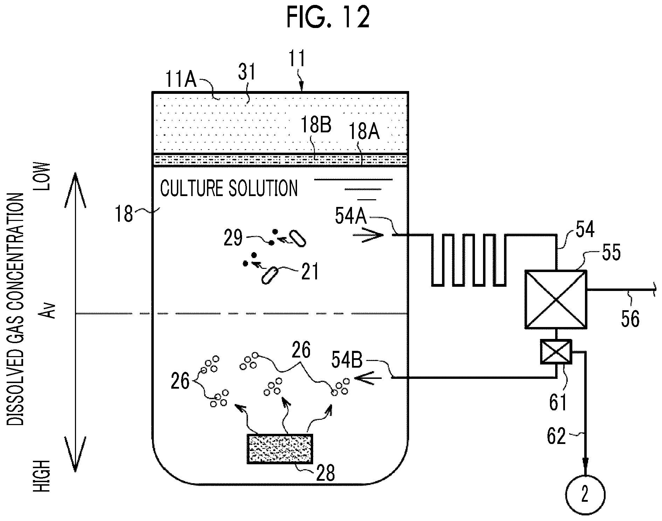

In addition, as shown in Fig. 12, the culture solution circulation path 54 has an extraction port 54A for extracting the culture solution 18 from the culture tank 11, and a return port 54B for returning a part of the extracted culture solution 18 to the culture tank 11. In the culture solution 18 in the culture tank 11, the dissolved gas concentration of the raw material gas 26 is low on an upper side in a height direction, and the dissolved gas concentration is high on a lower side. The extraction port 54A is set in a low-concentration region in which the dissolved gas concentration is relatively low, and the return port 54B is set in a high-concentration region in which the dissolved gas concentration is relatively high. More specifically, the extraction port 54A is set in a low-concentration region in which the dissolved gas concentration of the culture solution 18 in the culture tank 11 is lower than an average value Av.

As a result, in a case where a part of the culture solution 18 is extracted from the culture tank 11 in order to circulate the culture solution 18, it is easy to eliminate a concentration unevenness of the dissolved gas concentration of the culture solution 18 in the culture tank 11.

### (Stirring of Culture tank, Temperature Adjustment, and Sensing of Various Types of Information)

In addition, in Fig. 8, the stirring rod 41 that stirs the culture solution 18 is provided in the culture tank 11. The stirring rod 41 is a support rod provided with a screw 41A, and is provided such that the screw 41A is disposed in the culture solution 18. The stirring rod 41 rotates with the support rod as a rotation shaft by driving a motor 42 controlled by the processor 17. As a result, the culture solution 18 is stirred. The stirring rod 41 stirs the culture solution 18 to diffuse the hydrogen-oxidizing bacteria 21 and the raw material gas 26 in the culture solution 18 such that local uneven distribution does not occur.

In addition, the stirring rod 41 stirs the culture solution 18 to generate bubbles from the culture solution 18 and form the above-described foam layer 18B on the liquid surface 18A of the culture solution 18.

In addition, the culture tank 11 is provided with the thermometer 46, the temperature adjuster 47, the pressure gauge 48, the dissolved gas concentration measurement unit 49, and a density measurement unit 50. The thermometer 46 measures the temperature T in the culture solution 18. The temperature adjuster 47 heats or cools the culture solution 18 in the culture tank 11. The pressure gauge 48 measures the pressure PS in the space 11A. The dissolved gas concentration measurement unit 49 measures the dissolved gas concentration Dr of the raw material gas 26 dissolved in the culture solution 18. The density measurement unit 50 measures a density ρ of the hydrogen-oxidizing bacteria 21 present in the culture solution 18 in the culture tank 11.

The culture solution 18 is maintained at a target temperature suitable for culturing the hydrogen-oxidizing bacteria 21. The target temperature is, for example, 40°C or higher and 95°C or lower. The target temperature is set for each microorganism. In a case where the hydrogen-oxidizing bacteria 21 belongs to the genus Hydrogenophilus, the target temperature is more preferably about 50°C. The culture solution 18 is heated to the target temperature, and in some cases, cooling is required. This is because the culture solution 18 may exceed the target temperature due to heat generation during the proliferation of the microorganisms to be cultured. The processor 17 controls driving of the temperature adjuster 47 such that the temperature T of the culture solution 18 is maintained at the target temperature, based on the temperature T measured by the thermometer 46.

In addition, the processor 17 controls a pressure of the mixed gas 31 in the space 11A based on the pressure PS measured by the pressure gauge 48. By setting the pressure PS of the mixed gas 31 to a target pressure, solubility of the raw material gas 26 in the culture solution 18 is improved. In this way, the processor 17 controls the pressure of the mixed gas 31 to ensure appropriate solubility in the culture solution 18. As a result, culture efficiency of the hydrogen-oxidizing bacteria 21 is improved. An adjustment target of the pressure PS of the mixed gas 31 is at least one of the supply amount of the raw material gas 26, the discharge amount of the mixed gas 31, or the supply amount of the adjustment gas 33. The processor 17 controls the driving of each pump 22 to adjust the supply amount or the discharge amount of the gas.

More specifically, the dissolved gas concentration measurement unit 49 measures the concentration of each component (hydrogen, oxygen, and carbon dioxide) contained in the raw material gas 26 dissolved in the culture solution 18. As an example, the dissolved gas concentration measurement unit 49 is composed of a dissolved hydrogen meter (for example, model number KM2100DH manufactured by Kyoei Denshi Kenkyusho) that measures a dissolved hydrogen concentration (Dr(H₂)), a dissolved oxygen meter (for example, model number InPro6860i manufactured by Mettle Toledo) that measures a dissolved oxygen concentration (Dr(O₂)), and a dissolved carbon dioxide meter (for example, model number InPro5000i manufactured by Mettle Toledo) that measures a dissolved carbon dioxide concentration (Dr(CO₂)). The dissolved gas concentration measurement unit 49 outputs measured values to the processor 17.

As shown in Fig. 9, the raw material gas tank 12 accommodates each component (hydrogen, oxygen, and carbon dioxide) of the raw material gas 26. In addition, the supply path 12A and the pump 22 are provided for each component.

The processor 17 acquires a measured value of the dissolved gas concentration for each component from the dissolved gas concentration measurement unit 49, compares the dissolved gas concentration with the target value for each component, and supplies the component that is deficient in the raw material gas 26 to the culture tank 11. Through such control of a supply amount of each component of the raw material gas 26, the processor 17 can maintain the dissolved gas concentration of the raw material gas 26 in the culture solution 18 at an appropriate value.

The density measurement unit 50 measures a density ρ of a culture target present in the culture solution 18 in the culture tank 11. The density measurement unit 50 is composed of, for example, a total cell density sensor (for example, model number: Dencytee RS485 manufactured by Hamilton). In the present example, since the culture target is the hydrogen-oxidizing bacteria 21, the density measurement unit 50 measures the density ρ of the hydrogen-oxidizing bacteria 21 in the culture solution 18. The density ρ of the hydrogen-oxidizing bacteria 21 has a positive correlation with a consumption amount of the raw material gas 26 consumed by the hydrogen-oxidizing bacteria 21. Measuring the density ρ can be used, for example, to calculate the consumption amount of the raw material gas 26 consumed by the hydrogen-oxidizing bacteria 21, or for a process check to verify whether or not the consumption amount of the raw material gas 26 is appropriate with respect to the amount of the hydrogen-oxidizing bacteria 21.

A water level gauge 66 measures a water level of the culture solution 18 in the culture tank 11, that is, a height of the liquid surface 18A. An amount of the culture solution 18 present in the culture tank 11 can be ascertained by the water level of the culture solution 18 measured by the water level gauge 66. The water level gauge 66 is used for a process check of whether or not the amount of the culture solution 18 is appropriate, calculation of a necessary replenishment amount of the culture solution 18, and the like.

In addition, the culture tank 11 is provided with an explosion vent 67. The explosion vent 67 is configured to be destroyed earlier than other portions in a case where an explosion occurs in the culture tank 11, thereby safely releasing the pressure inside the culture tank 11 and preventing the damage from spreading. A place where large damage does not occur even in a case where the pressure is released, or a place where measures are taken so that damage does not spread is selected as the vicinity of the explosion vent 67.

The processor 17 is configured of, for example, a central processing unit (CPU) and a random access memory (RAM), and controls each unit of the culture device 10 as described above. In addition, the memory 17A is a non-volatile memory in which various types of setting information are stored, such as a flash memory.

### (Control of Bubble Diameter of Raw Material Gas Supplied to Culture Tank)

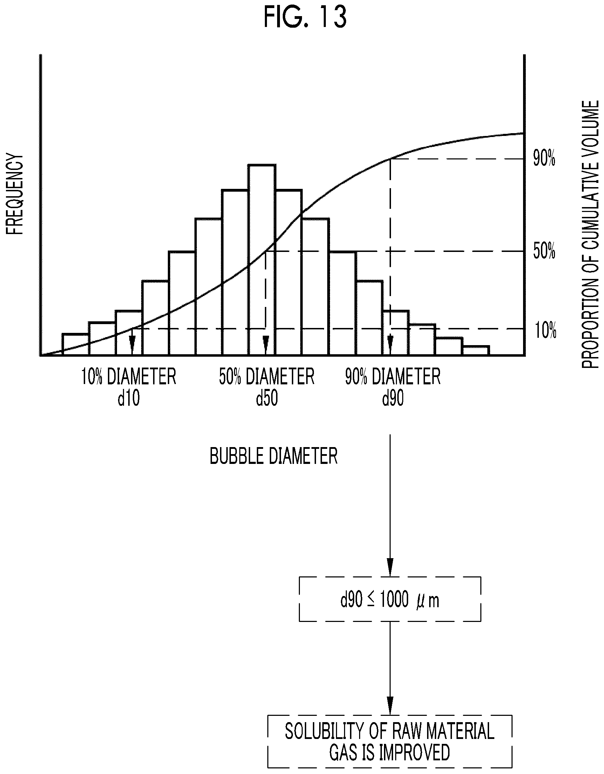

For a bubble diameter of the raw material gas 26 generated in the culture solution 18 of the culture tank 11, d90 is set to 1000 µm or less. As shown in Fig. 13, d90 refers to a bubble diameter at which a cumulative volume of bubbles is 90% of a total volume of all the bubbles in a case where volumes of all the bubbles are sequentially integrated from a bubble with the smallest diameter. A triangular distribution in a bar graph shown in Fig. 13 is a histogram of bubble diameters of the bubbles generated in the culture solution 18, in which a horizontal axis is a bubble diameter and a vertical axis is a frequency. In a case where the volumes of such bubble diameters are cumulated, a line graph sloping upward to the right as shown in Fig. 13 is obtained. d90 is a bubble diameter at which a proportion of the cumulative volume is 90% of the total volume, and in the culture device 10, for example, this bubble diameter is set to be 1000 µm or less. Such a bubble diameter is determined by, for example, specifications of a bubble generator such as the sparger 28. Regarding the bubble diameter, by setting d90 to 1000 µm or less, the solubility of the raw material gas 26 in the culture solution 18 can be improved. A more preferable bubble diameter is that d90 is 500 µm or less, and it is still more preferable that d90 is 200 µm or less. By setting the bubble diameter to such a value, it is possible to ensure high solubility of the raw material gas 26 even under conditions in which the raw material gas 26 is difficult to dissolve, for example, in a case where the temperature of the culture solution 18 is high or in a case where a depth of the culture tank 11 is shallow.

### (Surface Tension Depressant)

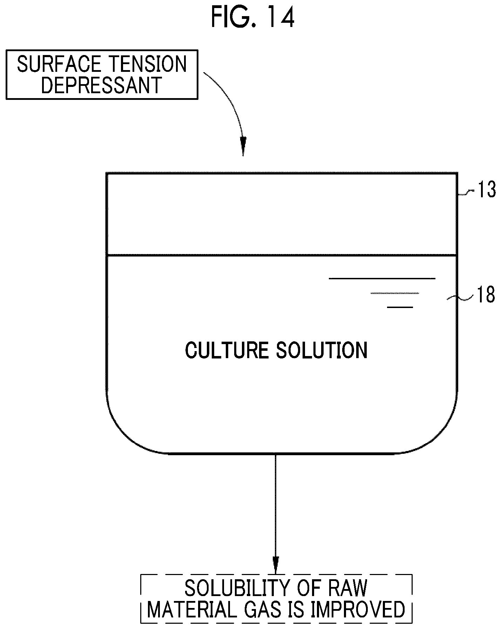

In addition, as shown in Fig. 14, the culture solution 18 may contain a surface tension depressant by adding the surface tension depressant. In a case where a surface tension of the culture solution 18 is reduced, the diameter of the bubble is likely to be reduced, and thus the solubility of the raw material gas 26 containing hydrogen, which is a flammable gas, can be improved. The surface tension of the culture solution 18 is preferably 65 mN/m or less. With such a surface tension, it is easy to set d90 of the bubble diameter to 1000 µm or less. An addition amount of the surface tension depressant is determined such that the surface tension is set to this value. The surface tension of the culture solution 18 is more preferably 55 mN/m or less and still more preferably 45 mN/m or less. By setting the surface tension to such a value, it is easy to reduce the bubble diameter.

### (Reuse of Waste Gas)

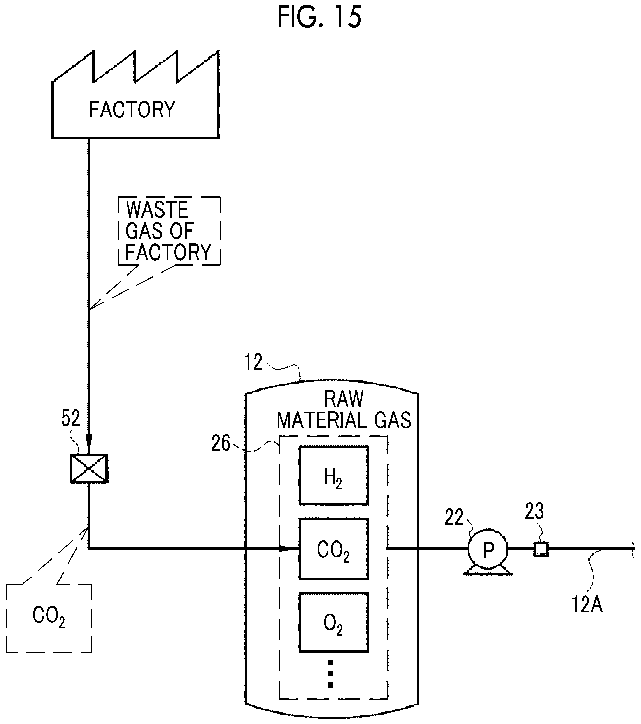

In addition, as shown in Fig. 15, the culture device 10 may reuse a waste gas containing carbon dioxide discharged from an external device as the raw material gas 26 of the culture device 10. The external device is a device other than the culture device 10, and may be, for example, another device in the same facility as a facility in which the culture device 10 operates, or another device in another facility (factory or the like). In a case where the waste gas is reused, the component separation mechanism 52 separates carbon dioxide from the waste gas, and the separated carbon dioxide is reused as the raw material gas 26. In this way, by reusing carbon dioxide contained in the waste gas, it is possible to contribute to the reduction of the carbon dioxide emissions.

In the above example, the hydrogen-oxidizing bacteria 21 has been described as an example of microorganisms, but the technology of the present disclosure can also be applied to microorganisms other than the hydrogen-oxidizing bacteria 21 or cells. As the microorganisms, for example, acetogenic bacteria such as Clostridium autoethanogenum, or methanotrophic bacteria such as Methylococcus capsulatus may be used. In a case of the acetogenic bacteria, the components of the raw material gas 26 are hydrogen (H₂), carbon monoxide (CO), and carbon dioxide (CO₂), which are flammable gases. In a case of the methanotrophic bacteria, the components of the raw material gas 26 are methane (CH₄), which is a flammable gas, and air. In a case of the methanotrophic bacteria, the components of the raw material gas 26 are methane (CH4), which is a flammable gas, and air. In addition, the cell is a morphological and/or functional unit constituting a living body, and the living body from which the cell originates is not particularly limited, but may be an animal or a plant. The cell may be an established cell or a genetically modified cell, and may include an artificial cell. Examples of the cells include Chinese hamster ovary cells (CHO cells), human embryonic kidney cells 293 (HEK293 cells), and Spodoptera frugiperda cells 9 (Sf9 cells).

The processor described above is not limited to a general-purpose processor such as a CPU that functions as various processing units by executing software (program), and includes a programmable logic device (PLD) which is a processor capable of changing a circuit configuration after manufacture such as a field programmable gate array (FPGA), a dedicated electric circuit which is a processor having a circuit configuration exclusively designed to execute specific processing such as an application specific integrated circuit (ASIC), and the like.

Furthermore, as a hardware structure of the various processors, more specifically, an electric circuitry in which circuit elements such as semiconductor elements are combined can be used.

The above-described contents and illustrated contents are detailed descriptions of parts related to the technology of the present disclosure, and are merely examples of the technology of the present disclosure. For example, the above descriptions related to configurations, functions, operations, and advantageous effects are descriptions related to examples of configurations, functions, operations, and advantageous effects of the parts related to the technology of the present disclosure. Therefore, it is needless to say that unnecessary parts may be deleted, or new elements may be added or replaced with respect to the above-described contents and illustrated contents within a scope not departing from the spirit of the technology of the present disclosure. In order to avoid complications and easily understand the parts according to the technology of the present disclosure, in the above-described contents and illustrated contents, common technical knowledge and the like that do not need to be described to implement the technology of the present disclosure are not described.

The disclosure of JP2022-140319 filed on September 2, 2022 and the disclosure of JP2023-125055 filed on July 31, 2023 are incorporated in their entirety in the present specification by reference. In addition, all documents, patent applications, and technical standards described in the present specification are incorporated in the present specification by reference to the same extent as in a case where each document, patent application, and technical standard are specifically and individually noted to be incorporated by reference.

Furthermore, the following appendices will be disclosed in relation to the above-described embodiments.

### <Appendix 1>

A culture device for culturing a microorganism or a cell that uses a raw material gas containing a flammable gas for proliferation or for production of an organic substance, the culture device comprising: a culture tank that accommodates a culture solution in which the microorganism or the cell is cultured; a raw material gas supply unit that supplies the raw material gas to the culture tank; a composition ratio measurement unit that measures a composition ratio of a mixed gas in a space present above a liquid surface of the culture solution in the culture tank; and a composition ratio control mechanism that supplies an adjustment gas to the space according to the measured composition ratio of the mixed gas, thereby maintaining the composition ratio of the mixed gas in the space at a target value outside a preset explosion range.

### <Appendix 2>

The culture device according to Appendix 1, in which the adjustment gas is an inert gas.

### <Appendix 3>

The culture device according to Appendix 1 or 2, in which the target value outside the explosion range is set to an oxygen concentration of less than 5% or a hydrogen concentration of less than 4%, and the composition ratio control mechanism controls the composition ratio based on the target value.

### <Appendix 4>

The culture device according to any one of Appendices 1 to 3, in which the target value outside the explosion range is an oxygen concentration of 5% or more and 9% or less and a water vapor concentration of 7% or more, and the composition ratio control mechanism controls the composition ratio based on the target value.

### <Appendix 5>

The culture device according to any one of Appendices 1 to 4, further comprising a temperature control mechanism that controls a temperature of the culture solution.

### <Appendix 6>

The culture device according to Appendix 5 citing Appendix 4, in which the temperature control mechanism controls the temperature of the culture solution to 40°C or higher.

### <Appendix 7>

The culture device according to any one of Appendices 1 to 6, further comprising a scattering mechanism that scatters a liquid containing water into the space.

### <Appendix 8>

The culture device according to Appendix 7, in which the liquid is an aqueous solution containing at least a part of components of the culture solution.

### <Appendix 9>

The culture device according to any one of Appendices 1 to 8, further comprising a foam layer forming portion that forms a foam layer on the liquid surface of the culture solution.

### <Appendix 10>

The culture device according to any one of Appendices 1 to 9, further comprising a circulation path for reusing the mixed gas in the space as the raw material gas.

### <Appendix 11>

The culture device according to any one of Appendices 1 to 10, in which the flammable gas is hydrogen.

### <Appendix 12>

The culture device according to Appendix 11, in which the raw material gas contains hydrogen, oxygen, and carbon dioxide.

### <Appendix 13>

The culture device according to Appendix 12, in which the adjustment gas is carbon dioxide.

### <Appendix 14>

The culture device according to Appendix 13, in which the microorganism is a hydrogen-oxidizing bacterium.

### <Appendix 15>

The culture device according to any one of Appendices 1 to 14, further comprising: a pressure measurement unit that measures a pressure inside the space; and a pressure control mechanism that controls the pressure by adjusting at least one of a supply amount of the raw material gas, a discharge amount of the mixed gas, or a supply amount of the adjustment gas, based on the measured pressure.

### <Appendix 16>

The culture device according to any one of Appendices 1 to 15, further comprising: a dissolved gas concentration measurement unit that measures a dissolved gas concentration of the raw material gas dissolved in the culture solution; and a raw material gas control mechanism that controls a supply amount of the raw material gas according to the dissolved gas concentration.

### <Appendix 17>

The culture device according to Appendix 16, in which the dissolved gas concentration measurement unit measures a concentration of each component contained in the raw material gas, and the raw material gas control mechanism controls the supply amount for each component.

### <Appendix 18>

A method for controlling a culture device for culturing a microorganism or a cell that uses a raw material gas containing a flammable gas for proliferation or for production of an organic substance, the culture device including a culture tank that accommodates a culture solution in which the microorganism or the cell is cultured, and a raw material gas supply unit that supplies the raw material gas to the culture tank, the method comprising: measuring a composition ratio of a mixed gas in a space present above a liquid surface of the culture solution in the culture tank; and supplying an adjustment gas to the space according to the measured composition ratio of the mixed gas, thereby maintaining the composition ratio of the mixed gas in the space at a target value outside a preset explosion range.

## Claims

1. A culture device for culturing a microorganism or a cell that uses a raw material gas containing a flammable gas for proliferation or for production of an organic substance, the culture device comprising:
a culture tank that accommodates a culture solution in which the microorganism or the cell is cultured;
a raw material gas supply unit that supplies the raw material gas to the culture tank;
a composition ratio measurement unit that measures a composition ratio of a mixed gas in a space present above a liquid surface of the culture solution in the culture tank; and
a composition ratio control mechanism that supplies an adjustment gas to the space according to the measured composition ratio of the mixed gas, thereby maintaining the composition ratio of the mixed gas in the space at a target value outside a preset explosion range.

2. The culture device according to claim 1,
wherein the adjustment gas is an inert gas.

3. The culture device according to claim 1,
wherein the target value outside the explosion range is set to an oxygen concentration of less than 5% or a hydrogen concentration of less than 4%, and
the composition ratio control mechanism controls the composition ratio based on the target value.

4. The culture device according to claim 1,
wherein the target value outside the explosion range is an oxygen concentration of 5% or more and 9% or less and a water vapor concentration of 7% or more, and
the composition ratio control mechanism controls the composition ratio based on the target value.

5. The culture device according to claim 1, further comprising:
a temperature control mechanism that controls a temperature of the culture solution.

6. The culture device according to claim 4 or 5,
wherein the temperature control mechanism controls the temperature of the culture solution to 40°C or higher.

7. The culture device according to claim 1, further comprising:
a scattering mechanism that scatters a liquid containing water into the space.

8. The culture device according to claim 7,
wherein the liquid is an aqueous solution containing at least a part of components of the culture solution.

9. The culture device according to claim 1, further comprising:
a foam layer forming portion that forms a foam layer on the liquid surface of the culture solution.

10. The culture device according to claim 1, further comprising:
a circulation path for reusing the mixed gas in the space as the raw material gas.

11. The culture device according to claim 1,
wherein the flammable gas is hydrogen.

12. The culture device according to claim 11,
wherein the raw material gas contains hydrogen, oxygen, and carbon dioxide.

13. The culture device according to claim 12,
wherein the adjustment gas is carbon dioxide.

14. The culture device according to claim 13,
wherein the microorganism is a hydrogen-oxidizing bacterium.

15. The culture device according to claim 1, further comprising:
a pressure measurement unit that measures a pressure inside the space; and
a pressure control mechanism that controls the pressure by adjusting at least one of a supply amount of the raw material gas, a discharge amount of the mixed gas, or a supply amount of the adjustment gas, based on the measured pressure.

16. The culture device according to claim 1, further comprising:
a dissolved gas concentration measurement unit that measures a dissolved gas concentration of the raw material gas dissolved in the culture solution; and
a raw material gas control mechanism that controls a supply amount of the raw material gas according to the dissolved gas concentration.

17. The culture device according to claim 16,
wherein the dissolved gas concentration measurement unit measures a concentration of each component contained in the raw material gas, and
the raw material gas control mechanism controls the supply amount for each component.

18. A method for controlling a culture device for culturing a microorganism or a cell that uses a raw material gas containing a flammable gas for proliferation or for production of an organic substance, the culture device including a culture tank that accommodates a culture solution in which the microorganism or the cell is cultured, and a raw material gas supply unit that supplies the raw material gas to the culture tank, the method comprising:
measuring a composition ratio of a mixed gas in a space present above a liquid surface of the culture solution in the culture tank; and
supplying an adjustment gas to the space according to the measured composition ratio of the mixed gas, thereby maintaining the composition ratio of the mixed gas in the space at a target value outside a preset explosion range.
